(19) Europäisches Patentamt
European Patent Office
Office européen des brevets

(11) Numéro de publication: **0 130 876**
**B1**

# (12) FASCICULE DE BREVET EUROPEEN

(45) Date de publication du fascicule du brevet: **20.04.88**

(21) Numéro de dépôt: **84401231.0**

(22) Date de dépôt: **15.06.84**

(51) Int. Cl.[4]: **C 07 B 39/00** // C07C25/13, C07C125/03, C07C41/22, C07C149/34, C07C68/06, C07C39/26

(54) **Procédé d'halogénation et de fluoration simultanées de dérivés aromatiques.**

(30) Priorité: **23.06.83 FR 8310372**

(43) Date de publication de la demande: **09.01.85 Bulletin 85/2**

(45) Mention de la délivrance du brevet: **20.04.88 Bulletin 88/16**

(84) Etats contractants désignés: **AT BE CH DE FR GB IT LI LU NL SE**

(56) Documents cités:
**FR - A - 2 375 168**

**HOUBEN-WEYL: "Methoden der organischen Chemie", vol. V/3, Halogenverbindungen", 1962, page 679; Georg Thieme Verlag, Stuttgart (DE)**
**Friedel-Crafts and related reactions, George A. Olau, p. 269-270 (1963), Interscience publication**

(73) Titulaire: **RHONE-POULENC CHIMIE, 25, quai Paul Doumer, F-92408 Courbevoie Cédex (FR)**

(72) Inventeur: **Desbois, Michel, 526 Chemin du Bois, F-69140 Rillieux (FR)**
Inventeur: **Disdier, Camille, 54 Avenue Cabias, F-69004 Lyon (FR)**

(74) Mandataire: **Cazes, Jean-Marie et al, RHONE-POULENC INTERSERVICES Service Brevets Chimie 25, quai Paul Doumer, F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé d'halogénation et de fluoration simultanées de dérivés aromatiques substitués par au moins un groupe comportant un motif halogénoalkyle. Elle concerne plus particulièrement un procédé d'halogénation du noyau aromatique et simultanément un procédé de fluoration dudit groupe par échange halogène-fluor.

On entend par halogénation de dérivés aromatiques, la fixation d'au moins un atome de chlore ou de brome.

On entend par dérivés aromatiques substitués par au moins un groupe comportant un motif halogénoalkyle, tout dérivé aromatique mono ou polycyclique répondant à la formule générale suivante:

$$Ar\text{-}(A\text{-}CX_1X_2Y)_n \qquad (I)$$

dans laquelle:
Ar représente un radical aromatique mono ou polycyclique
A représente une liaison covalente, un atome d'oxygène ou un atome de soufre
$X_1$ et $X_2$ identiques ou différents représentent un halogène
Y correspond à l'hydrogène, à un halogène ou à une chaîne alkyle de 1 à 3 atomes de carbone éventuellement halogénée. Les halogènes correspondant à $X_1$, $X_2$ et Y sont identiques ou différents mais au moins l'un d'entre eux ne doit pas être le fluor.
n est égal à 1 ou 2 et de préférence égal à 1.

Pour plus de clarté, on désignera l'échange halogène-fluor du groupe comportant un motif halogénoalkyle par le terme fluoration-échange.

Il est connu depuis longtemps de procéder à une fluoration-échange en milieu acide fluorhydrique à partir de dérivés aromatiques substitués par au moins un groupe comportant un motif halogénoalkyle puis ensuite de procéder dans une deuxième étape indépendante à une chloration du dérivé aromatique, substitué par au moins un groupe comportant un motif fluoroalkyle, en présence d'un catalyseur de chloration tel que notamment $FeCl_3$, $BF_3$ (brevet allemand DE-A 825 397), Pt sur alumine (brevet allemand DE-A 1 034 609).

Le fait d'opérer en deux étapes présente de nombreux inconvénients et surtout provoque une perte de rendement.

Il a été possible tel que décrit dans Houben Weyl (vol. 3 p. 679) d'opérer la chloration et la fluoration-échange en une seule étape en utilisant un catalyseur tel que le pentachlorure d'antimoine dans l'acide fluorhydrique anhydre.

L'homme de l'art connait bien les propriétés de ce catalyseur qui se présente à la fois comme un catalyseur de chloration et comme un catalyseur de fluoration.

Le procédé précédemment décrit présente l'avantage d'opérer en une seule étape, mais il nécessite la présence d'un catalyseur, qui n'est pas recyclable ce qui du point de vue technique pose des problèmes de pollution et du point de vue économique pose des problèmes de rentabilité.

La présente invention qui remédie aux inconvénients de l'art antérieur concerne un procédé de traitement d'un dérivé aromatique perhalogénoalkylé consistant en une halogénation directe du noyau aromatique et en une fluoration du motif perhalogénoalkyle caractérisé en ce qu'on fait réagir le dérivé aromatique perhalogénoalkylé avec l'halogène moléculaire dans l'acide fluorhydrique liquide en l'absence de catalyseur porteur du même halogène que celui introduit par l'halogène moléculaire.

L'acide fluorhydrique joue une double fonction; il sert d'agent de fluoration du groupe substituant le noyau et sert également de solvant lors de l'halogénation du noyau aromatique.

On connaît dans l'art antérieur le brevet français n° 7 739 363 publié sous le n° FR-A 2 375 169 qui décrit la préparation de perfluoroalkylbenzènes. Selon ce procédé, on fait réagir l'alkylbenzène correspondant avec du chlore et de l'acide fluorhydrique en phase gazeuse à haute température. La température est comprise généralement entre 350 et 600°C. Il est précisé dans ce document qu'à partir d'une certaine température (supérieure à 450°C généralement) à côté de la réaction de fluoration de la chaîne alkyle latérale on assiste à des réactions de chloration du noyau benzénique.

Cette réaction de chloration du noyau benzénique apparaît donc comme résultant d'une réaction parallèle mettant en œuvre à une température supérieure à 450°C le chlore présent dans le milieu, l'acide fluorhydrique ne participant pas à la réaction. Ceci est confirmé à l'homme de l'art par le brevet de la République Démocratique Allemande DD-A 15 100 qui montre qu'à haute température lorsqu'on fait réagir, en l'absence d'acide fluorhydrique, du chlore sur un alkylbenzène, on obtient à côté du perchloroalkylbenzène des dérivés chlorés sur le noyau benzénique. Ceci est encore confirmé par l'analyse faite de ce brevet allemand dans le brevet des Etats-Unis d'Amérique US-A 3 816 287.

Ainsi donc, l'art antérieur montrait à l'homme de l'art que pour obtenir à partir d'un alkylbenzène le perfluoroalkylbenzène correspondant, il fallait mettre en œuvre, d'une part, du chlore (à haute température puisqu'on opérait sans catalyseur), chlore qui donnait à côté de la réaction de perchloration de la chaîne alkyle, des réactions secondaires de chloration sur le noyau et, d'autre part, de l'acide fluorhydrique pour remplacer les atomes de chlore de la chaîne perchloroalkyle par des atomes de fluor.

Il a été possible de réaliser, grâce à la présente invention, s'écartant très nettement de l'interprétation admise dans l'art antérieur, l'halogénation d'un noyau aromatique, d'une part, sans catalyseur et, d'autre part, sans être tenu d'opérer à haute température.

Ces objectifs sont atteints par la mise en œuvre

d'un halogène en présence d'acide fluorhydrique liquide.

Le présent procédé peut être utilisé pour halogéner le noyau aromatique et échanger les halogènes de la chaîne halogénoalkyle de tous les dérivés aromatiques substitués par un ou plusieurs groupes comportant un motif halogénoalkyle quels que soient les autres substituants présents sur le noyau.

En effet, le noyau aromatique peut comporter en plus du groupe comportant un motif halogénoalkyle par exemple au moins un halogène, un groupe hydroxyle, un groupe nitro, un groupe cyano, un groupe isocyanate, un groupe carboxylique, un groupe alkyle ou alkoxy ou un groupe phényle ou phénoxy.

La fixation de l'halogène sur le noyau se fera selon les règles de substitution bien connues de l'homme de l'art, en fonction de la présence de radicaux ortho, para ou méta orienteurs.

La fluoration-échange permet l'échange des halogènes des groupes halogénométhyles, halogénométhoxyles et halogénothiométhyles en $-CF_3-$, $-OCF_3$ et $-SCF_3$.

Dans le cas des groupes halogénoalkoxyles et halogénothioalkyles, l'échange se fera sur le carbone de la chaîne alkyle situé en a de l'hétéroatome.

Ainsi, les groupes $-OCCl_2-CCl_3$, $-SCCl_2CCl_3$, seront transformés après fluoration-échange en $-OCF_2$, $CCl_3$, $-SCF_2CCl_3$.

Par contre les atomes d'halogène directement fixés sur le noyau benzénique ne sont pas touchés par la fluoration-échange.

L'acide fluorhydrique utilisé pour la présente invention est de préference de l'acide fluorhydrique anhydre.

Le rapport molaire de l'acide fluorhydrique sur le composé aromatique de départ est de préférence compris entre 10 et 100. Une quantité nettement supérieure n'est pas nuisible à l'invention.

La quantité d'halogène mise en œuvre est fixée par l'homme de l'art compte-tenu du produit recherché correspondant à une mono ou polyhalogénation. Pour une monohalogénation, on opère de préférence en présence d'un défaut stœchiométrique d'halogène c'est-à-dire avec un rapport molaire halogène au composé aromatique compris de préférence entre 0,5 et 0,9. Pour une polyhalogénation, on préfère opérer avec un excès d'halogène. L'halogène peut être mis en œuvre dans une enceinte fermée sous pression autogène (généralement 1 à 50 bars) ou sous pression atmosphérique par barbotage ou dans tout autre dispositif connu de l'homme de l'art.

La température de mise en œuvre est de préférence comprise entre -20°C et 150°C.

Lorsque la température sera supérieure à 20°C, la réaction devra avoir lieu sous pression puisque l'acide fluorhydrique doit être liquide.

La durée de la réaction varie de quelques minutes à quelques heures.

Cette durée de réaction varie avec le nombre d'atomes d'halogène que l'on veut fixer sur le noyau ainsi qu'avec les matières premières de départ et avec la température de la réaction.

Le produit final halogéné aromatique est isolé par exemple par distillation de l'acide fluorhydrique qui ainsi est récupérable et recyclable ce qui est un avantage important du procédé de l'invention. Il peut aussi être isolé par extraction à l'aide de solvants organiques bien connus de l'homme de l'art.

On peut citer comme exemple de produits de formule (I): le trichlorométhylbenzène, le méta-bis-trichlorométhylbenzène, le trichlorométhoxybenzène, le parabistrichlorométhoxybenzène, le trichlorométhylthiobenzène, les chlorotrichlorométhylbenzènes, les fluorotrichlorométhylbenzènes, le dichlorobromométhylbenzène, le tribromométhylbenzène, les chlorotrichlorométhoxybenzènes, les fluorotrichlorométhoxybenzènes, le p-trichlorométhylphénylisocyanate, le chloroformiate de p-trichlorométhylphényle, le pentachloroéthoxybenzène, le pentachloroéthylthiobenzène, le paratrichlorométhylphénol, le chloro-2 trichlorométhyl-4 phénol.

Bien que l'invention ne soit pas limitée à ces composés, elle trouve une application particulièrement intéressante pour l'halogénation et la fluoration-échange des dérivés aromatiques perchloroalkylés, perchloroalkoxylés et perchlorothioalkylés comme par exemple les trichlorométhylbenzènes, les trichlorométhoxybenzènes, les trichlorométhylthiobenzènes.

Les dérivés halogénés aromatiques substitués par un groupe fluoroalkyle sont utilisés comme intermédiaires de synthèse dans l'industrie pharmaceutique et phytosanitaire.

La présente invention sera plus aisément comprise à l'aide des exemples suivants:

Exemple 1

Dans un réacteur de 250 ml, agité par un barreau aimanté et refroidi aux environs de 0°C, on introduit 50 ml (2,5 m) d'acide fluorhydrique anhydre et 9,8 g (0,05 m) de trichlorométhylbenzène. On laisse le mélange réactionnel se dégazer sous agitation (départ d'acide chlorhydrique) pendant une heure puis le réacteur est fermé et porté à la pression de 5,5 bars (à 20°C) par du chlore gazeux. On chauffe alors le tout, sous agitation, à 90°C pendant 4 heures. Après refroidissement de nouveau vers 0°C, le réacteur est décomprimé et le brut réactionnel obtenu coulé sur 110 g de glace pilée. Le mélange hétérogène issu de ce traitement est extrait par 300 $cm^3$ de chlorure de méthylène.

Après décantation, les phases organiques sont rassemblées.

L'ensemble est lavé par 2 fois 100 $cm^3$ d'eau permutée et séché. Les analyses effectuées par chromatographie en phase gazeuse (% aire) spectrométrie IR et spectrométrie de masse nous donnent le résultat suivant:

- Trifluorométhylbenzène: 26,8%
- m-chlorotrifluorométhylbenzène: 48,1%
- autres chlorotrifluorométhylbenzènes: 25,1%

Exemple 2

On procède de manière identique à l'exemple 1 avec les composés et conditions ci-dessous et en remplaçant le traitement du brut réactionnel à la glace par une extraction de ce brut à l'aide de 2 fois 100 $cm^3$ de tétrachlorure de carbone, le traitement de ces phases organiques étant poursuivi normalement.

- Acide fluorhydrique anhydre: 100 g (5 m)
- p-fluorotrichlorométhylbenzène: 10,7 g (0,05 m)
- Température: 80°C
- Pression de chlore: 4 bars à 20°C
- Durée: 21 heures

Les analyses effectuées par chromatographie en phase gazeuse (% aire), spectrométrie IR et spectrométrie de masse nous donnent le résultat suivant:

- fluorotrifluorométhylbenzène: 58,6%
- fluoro-4 chloro-3 trifluorométhylbenzène: 24,6%

Exemple 3

On procède de manière identique à l'exemple 1 avec les composés et conditions suivants:

- Acide fluorhydrique anhydre: 100 g (5 m)
- p-trichlorométhylphénylisocyanate: 23,6 g (0,1 m)
- Température: 20°C
- Pression de chlore: 4 bars à 20°C
- Durée: 20 heures 25 mn

Les analyses effectuées par chromatographie en phase gazeuse (% aire), spectrométrie IR et spectrométrie de masse nous donnent le résultat suivant:

- Fluorure de carbamoyle de la trifluorométhyl-4 chloro-2 aniline: 81%

Exemple 4

On procède de manière identique à l'exemple 1 avec les composés et conditions ci-dessous et en remplaçant le traitement du brut réactionnel à la glace par une distillation jusqu'à 80°C en pied sous pression atmosphérique de ce brut afin d'éliminer au maximum l'acide fluorhydrique solvant.

- Acide fluorhydrique anhydre: 100 g (5 m)
- Trichlorométhoxybenzène: 10,6 g (0,05 m)
- Température: 120°C
- Pression de chlore: 4 bars à 20°C
- Durée: 3 heures

Les analyses effectuées par chromatographie en phase gazeuse (% aire), spectrométrie IR et spectrométrie de masse nous donnent le résultat suivant:

- p-chlorotrifluorométhoxybenzène: 53%
- dichlorotrifluorométhoxybenzène: 15%

Exemple 5

On procède de manière identique à l'exemple 1 avec les composés et conditions suivants:

- Acide fluorhydrique anhydre: 100 g (5 m)
- p-chlorotrichlorométhoxybenzène: 12,3 g (0,05 m)
- Température: 150°C
- Pression de chlore: 4 bars à 20°C
- Duréee: 3 heures 30 mn

Les analyses effectuées par chromatographie en phase gazeuse (% aire), spectrométrie IR et spectrométrie de masse nous donnent le résultat suivant:

- p-chlorotrifluorométhoxybenzène: 19%
- dichloro-2,4 trifluorométhoxybenzène: 28,3%
- Trichlorotrifluorométhoxybenzène: 12,5%

Exemple 6

On procède de manière identique à l'exemple 1, en remplaçant le chlore par le brome et avec les composés et conditions suivants:

- Acide fluorhydrique anhydre: 100 g (5 m)
- Trichlorométhylbenzène: 19,5 g (0,1 m)
- Température: 100°C
- Brome: 16 g (0,1 m)
- Durée: 24 heures

Les analyses effectuées par chromatographie en phase gazeuse (% aire), spectrométrie IR et spectrométrie de masse nous donnent le résultat suivant:

- Trifluorométhylbenzène: 36,4%
- m-bromotrifluorométhylbenzène: 57,7%

Exemple 7

On procède de manière identique à l'exemple 1, en remplaçant le chlore par le brome et avec les composés et conditions suivants:

- Acide fluorhydrique anhydre: 50 g (2,5 m)
- Trichlorométhylthiobenzène: 11,3 g (0,05 m)
- Température: 100°C
- Brome: 8 g (0,05 m)
- Durée: 3 heures 40 mn

Les analyses effectuées par chromatographie en phase gazeuse (% aire), spectrométrie IR et spectrométrie de masse nous donnent le résultat suivant:

- Trifluorométhylthiobenzène: 28%
- p-bromotrifluorométhylthiobenzène: 53,1%
- o-bromotrifluorométhylthiobenzène: 14,9%

Exemple 8

On procède de manière identique à l'exemple 1, en remplaçant le chlore par le brome et avec les composés et conditions suivants:

- Acide fluorhydrique anhydre: 100 g (5 m)
- Trichlorométhoxybenzène: 21,2 g (0,1 m)
- Température: 120°C
- Brome: 16 g (0,1 m)
- Durée: 3 heures 40 mn

Les analyses effectuées par chromatographie en phase gazeuse (% aire), spectrométrie IR et spectrométrie de masse nous donnent le résultat suivant:

- Trifluorométhoxybenzène: 14,1%
- monobromotrifluorométhoxybenzène: 55,6%

Exemple 9

On procède de manière identique à l'exemple 1 mais à pression atmosphérique et introduction du chlore par bullage au sein du milieu réactionnel et avec les composés et conditions suivants:

- Acide fluorhydrique anhydre: 40 g (2 m)

- p-trichlorométhylphénylisocyanate: 47,2 g (0,02 m)
- Température: 10°C
- Pression de chlore: p. atmosphérique
- Durée: 5 heures

Les analyses effectuées par chromatographie en phase gazeuse (% aire), spectrométrie IR et spectrométrie de masse nous donnent le résultat suivant:
- Fluorure de carbamoyle de la trifluorométhyl-4 chloro-2 aniline: 73%

Exemple 10

On procède de manière identique à l'exemple I avec les composés et conditions suivants:
- Acide fluorhydrique anhydre: 100 g (5 m)
- p-chlorotrichlorométhylbenzène: 23 g (0,1 m)
- Température: 100°C
- Pression de chlore: 4 bars à 20°C
- Durée: 18 heures

Les analyses effectuées par chromatographie en phase gazeuse (% aire), spectrométrie IR et spectrométrie de masse nous donnent le résultat suivant:
- p-chlorotrifluormethylbenzène: 21%
- Dichloro-3,4 trifluorométhylbenzène: 74%
- Autres chlorotrifluorométhylbenzènes: 5%

Exemple 11

On procède de manière identique à l'exemple 1 avec les composés et conditions suivants:
- Acide fluorhydrique anhydre: 100 g (5 m)
- p-bistrichlorométhoxybenzène: 17,25 g (0,05 m)
- Température: 150°C
- Pression de chlore: 4 bars à 20°C
- Durée: 4 heures

Les analyses effectuées par chromatographie en phase gazeuse (% aire), spectrométrie IR et spectrométrie de masse nous donnent le résultat suivant:
- p-bistrifluorométhoxybenzène: 42%
- monochloro p-bistrifluorométhoxybenzène: 14%
- dichloro-p-bistrifluorométhoxybenzène: 6%

Exemple 12

On procède de manière identique à l'exemple 1 avec les composés et conditions suivants:
- Acide fluorhydrique anhydre: 50 g (2,5 m)
- Chloroformiate de p-trichlorométhylphényle: 13,7 g (0,05 m)
- Température: 80°C
- Pression de chlore: 4 bars à 20°C
- Durée: 4 H 30

Les analyses effectuées par chromatographie en phase gazeuse (% aire), spectrométrie IR et spectrométrie de masse nous donnent le résultat suivant:
- fluoroformiate de p-trifluorométhylphényle: 70%
- fluoroformiate de chloro-2 trifluorométhyl-4 phényle: 1,5%

Exemple 13

On procède de manière identique à l'exemple 1 avec les composés et conditions suivants:
- Acide fluorhydrique anhydre: 50 g (2,5 m)
- Chloro-2 trichlorométhyl-4 phénol: 9,8 g (0,05 m)
- Température: 100°C
- Pression de chlore: 3 bars à 20°C
- Durée: 4 heures

Les analyses effectuées par chromatographie en phase gazeuse (% aire), spectrométrie IR et spectrométrie de masse nous donnent le résultat suivant:
- dichloro-2,6 et dichloro-2,5 trifluorométhylphénol: 75%
- trichlorotrifluorométhylphénol: 13%

**Revendications**

1. Procédé de traitement d'un dérivé aromatique perhalogénoalkylé consistant en une halogénation directe du noyau aromatique et en une fluoration du motif perhalogénoalkyle caractérisé en ce qu'on fait réagir le dérivé aromatique perhalogénoalkylé avec l'halogène moléculaire dans l'acide fluorhydrique liquide en l'absence de catalyseur porteur du même halogène que celui introduit par l'halogène moléculaire.

2. Procédé selon la revendication 1 caractérisé en ce que le dérivé aromatique substitué par au moins un groupe comportant un motif halogénoalkylé répond à la formule générale:
$Ar\text{-}(A\text{-}CX_1X_2Y)_n$ dans laquelle:
Ar représente un radical aromatique mono ou polycyclique
A correspond à une liaison covalente, à l'oxygène ou au soufre
$X_1$ et $X_2$ identiques ou différents représentent un halogène
Y correspond à l'un des groupes suivants: l'hydrogène, un halogène, une chaîne alkyle de 1 à 3 atomes de carbone éventuellement halogénée
les halogènes correspondant à $X_1$ $X_2$ et à Y étant identiques ou différents mais au moins l'un d'entre eux n'étant pas du fluor.
n est égal à 1 ou 2

3. Procédé selon la revendication 1 caractérisé en ce que n est égal à 1.

4. Procédé selon la revendication 1 caractérisé en ce que l'halogène est choisie parmi le chlore ou le brome.

5. Procédé selon la revendication 1 caractérisé en ce que l'acide fluorhydrique est anhydre.

6. Procédé selon les revendication 1 et 2 caractérisé en ce que l'acide fluorhydrique est utilisé selon un rapport molaire acide fluorhydrique au dérivé aromatique compris entre 10 et 100.

7. Procédé selon l'une quelconque des revendications précédentes caractérisé en ce que l'halogénation et la fluoration s'effectuent entre -20° et 150°C.

8. Procédé selon la revendication 1 ou 2 caractérisé en ce que les dérivés aromatiques sont

choisis parmi les dérivés perchloroalkylés, perchloroalkoxylés, perchlorothioalkylés.

9. Procédé selon la revendication 8 caractérisé en ce que les dérivés aromatiques sont choisis parmi les trichlorométhylbenzènes, trichlorométhoxybenzènes, trichlorométhylthiobenzènes.

## Patentansprüche

1. Verfahren zur Behandlung einer aromatischen Perhalogenalkylverbindung bestehend aus einer direkten Halogenierung des aromatischen Kerns und einer Fluorierung der Perhalogenalkylfunktion, dadurch gekennzeichnet, dass man die aromatische Perhalogenalkylverbindung mit molekularem Halogen in flüssiger Fluorwasserstoffsäure in Abwesenheit eines Katalysators der dasselbe Halogen aufweist, das durch molekulares Halogen eingeführt wird, reagieren lässt.

2. Verfahren nach Anspruch 1, dadurch gekennzeichnet, dass die aromatische Verbindung, die mit wenigstens einem Rest substituiert ist, der eine Halogenalkylfunktion enthält, der allgemeinen Formel entspricht:

$$Ar\text{-}(A\text{-}CX_1X_2Y)_n$$

in der:
Ar einen mono- oder polycyclischen aromatischen Rest darstellt
A einer covalenten Bindung, Sauerstoff oder Schwefel entspricht
$X_1$ und $X_2$ identisch oder verschieden sind und jeweils ein Halogen darstellen
Y einer der nachfolgenden Gruppen entspricht: Wasserstoff, einem Halogen, einer Alkylkette mit 1 bis 3 Kohlenstoffatomen, gegebenenfalls halogeniert,
wobei die Halogene, die $X_1$ $X_2$ und Y entsprechen identisch oder verschieden sind aber wenigstens eines von ihnen nicht Fluor ist und n gleich 1 oder 2 ist.

3. Verfahren nach Anspruch 1, dadurch gekennzeichnet, dass n gleich 1 ist.

4. Verfahren nach Anspruch 1, dadurch gekennzeichnet, dass das Halogen ausgewählt wird aus Chlor oder Brom.

5. Verfahren nach Anspruch 1, dadurch gekennzeichnet, dass die Fluorwasserstoffsäure wasserfrei ist.

6. Verfahren nach den Ansprüchen 1 und 2, dadurch gekennzeichnet, dass die Fluorwasserstoffsäure in einem molaren Verhältnis Fluorwasserstoffsäure zu aromatischer Verbindung zwischen 10 und 100 eingesetzt wird.

7. Verfahren nach einem der vorhergehenden Ansprüche, dadurch gekennzeichnet, dass die Halogenierung und die Fluorierung zwischen -20° und 150°C durchgeführt werden.

8. Verfahren nach Anspruch 1 oder 2, dadurch gekennzeichnet, dass die aromatischen Verbindungen ausgewählt werden aus den Perchloralkyl-, Perchloralkoxyl-, Perchlorthioalkylverbindungen.

9. Verfahren nach Anspruch 8, dadurch gekennzeichnet, dass die aromatischen Verbindungen ausgewählt werden aus den Trichlormethylbenzolen, Trichlormethoxybenzolen, Trichlormethylthiobenzolen.

## Claims

1. Process for the treatment of a perhaloalkyl aromatic derivative, consisting of a direct halogenation of the aromatic ring and of a fluorination of the perhaloalkyl unit, characterized in that the perhaloalkyl aromatic derivative is reacted with molecular halogen in liquid hydrofluoric acid in the absence of a catalyst carrying the same halogen as that introduced by the molecular halogen.

2. Process according to Claim 1, characterized in that the aromatic derivative substituted by at least one group containing a haloalkyl unit corresponds to the general formula:

$$Ar\text{-}(A\text{-}CX_1X_2Y)_n$$

in which
Ar represents a mono- or polycyclic aromatic radical
A corresponds to a covalent bond, to oxygen or to sulphur
$X_1$ and $X_2$, which may be identical or different, represent a halogen
Y corresponds to one of the following groups: hydrogen, a halogen, an optionally halogenated alkyl chain containing from1 to 3 carbon atoms the halogens corresponding to $X_1$, $X_2$ and Y being identical or different, but at least one of them not being fluorine
n is equal to 1 or 2.

3. Process according to Claim 1, characterized in that n is equal to 1.

4. Process according to Claim 1, characterized in that the halogen is chosen from amongst chlorine or bromine.

5. Process according to Claim 1, characterized in that the hydrofluoric acid is anhydrous.

6. Process according to Claims 1 and 2, characterized in that the hydrofluoric acid is employed according to a molar ratio of hydrofluoric acid to the aromatic derivative of between 10 and 100.

7. Process according to any one of the preceding claims, characterized in that the halogenation and the fluorination are carried out between -20° and 150°C.

8. Process according to Claim 1 or 2, characterized in that the aromatic derivatives are chosen from amongst perchloroalkyl, perchloroalkoxyl or perchlorothioalkyl derivatives.

9. Process according to Claim 8, characterized in that the aromatic derivatives are chosen from amongst trichloromethylbenzenes, trichloromethoxybenzenes or trichloromethylthiobenzenes.